# EUROPEAN PATENT APPLICATION

(11) **EP 4 847 004 A1**
(43) Date of publication of application: **02.12.2026**
(21) Application number: 25179834.4
(22) Date of filing: 30.05.2025
(51) Int. Cl.: A61K 8/22, A61K 8/23, A61K 8/24, A61K 8/34, A61K 8/41, A61K 8/49, A61Q 5/10

(54) **OXIDATIVE HAIR DYEING COMPOSITION, METHOD, KIT, AND USE**

(71) Applicant: Kao Corporation, Chuo-ku, Tokyo 103-8210 (JP)
(72) Inventor: MÖHRING, Hartmut, 64297 Darmstadt (DE)
(74) Representative: Hoffmann Eitle

(57) **Abstract**

An oxidative hair dyeing composition A comprising a) one or more organic or inorganic salt(s) yielding an acidic solution at a concentration of 1% by weight in water at 25°C under atmospheric pressure, b) one or more oxidative hair dye(s), wherein the composition A comprises water at 10% by weight or less, calculated to the total weight of composition A.

## Description

### Field of the invention

The present invention relates to an oxidative hair dyeing composition, kit, method of dyeing, and use of compounds.

### Background of the invention

Many people desire hair colors that are different from their natural hair color. There are at least three classes of compounds commonly used for hair coloring: 1) oxidative dyes, 2) direct dyes, and 3) pigments. In oxidative dyeing, colorless molecules are applied to the hair, penetrate the hair fiber due to their small molecular weight, and then polymerize in the presence of an oxidizing agent to form larger molecules that cannot be easily washed out. Direct dyes are colored compounds that are applied to the hair and color the hair according to its inherent color. Pigments are water-insoluble compounds that are commonly used in temporary hair coloration because they are deposited on the hair surface and are easily washed off.

There are many known product forms of oxidative hair dyes, the majority of which are aqueous. Like any other industry, the cosmetics industry is developing more and more products with improved or equivalent performance, but with a smaller environmental footprint. In recent years, solid formulations of oxidative hair dyes have received increasing attention because they are water-depleted, allow for a reduced product weight and therefore result in less carbon dioxide emissions during production, packaging, storage, and shipping.

One of the challenges with solid oxidative dye blends is their decreasing dyeing intensity upon storage. The oxidative dyes tend to polymerize prematurely during storage, resulting in a reduced coloring effect on the consumer's hair.

Therefore, oxidative dyes need to be stabilized to maintain their dyeing intensity, especially in water-depleted product forms. Several stabilization approaches are known, but there is still a need for better solutions.

### Summary of the invention

Therefore, the first object of the present invention is an oxidative hair dyeing composition A comprising:
a) One or more organic or inorganic salt(s) yielding an acidic solution at a concentration of 1% by weight in water at 25°C under atmospheric pressure,
b) One or more oxidative hair dye(s),

Wherein the composition A comprises water at 10% by weight or less, calculated to the total weight of composition A.

The second object of the present invention is a kit-of-parts comprising:
- the composition as defined above, and
- an aqueous composition B having a pH in the range of 1 to 6 and comprising one or more oxidizing agent(s), preferably hydrogen peroxide.

The third object of the present invention is a method for dyeing keratin fibers, preferably human keratin fibers, more preferably human hair, comprising the steps of:
(i) mixing the composition as defined above with a second aqueous composition as defined above to yield a ready-to-use composition having a pH in the range of 6 to 12,
(ii) applying the ready-to-use composition onto keratin fibers and leaving it for a time period in the range of 1 min to 60 min,
(iii) rinsing-off the keratin fibers and optionally shampooing the keratin fibers.

The fourth object of the present invention is the use of organic or inorganic salt(s) yielding an acidic solution at a concentration of 1% by weight in water at 25°C under atmospheric pressure as defined above for improving the storage stability of oxidative hair dyeing compositions comprising water at 10% by weight or less, calculated to the total weight of the oxidative hair dyeing composition.

### Detailed description of the invention

The inventors of the present invention have surprisingly and unexpectedly found that the composition, the kit, the methods, and the use according to the independent claims improved the dyeing intensity of oxidative dyes upon storage, increased sustainability, and increased customer satisfaction.

### Oxidative Hair Dyeing Composition A

The present invention is directed to an oxidative hair dyeing composition A comprising:
a) One or more organic or inorganic salt(s) yielding an acidic solution at a concentration of 1% by weight in water at 25°C under atmospheric pressure,
b) One or more oxidative hair dye(s),

Wherein the composition A comprises water at 10% by weight or less, calculated to the total weight of composition A.

The one or more compound(s) according to group a) are organic or inorganic salts yielding an acidic solution at a concentration of 1% by weight in water at 25°C under atmospheric pressure. The acidic pH is understood as commonly known in chemistry, i.e., the salts yield a pH below 7 in water. The pH thereby may be measured with a calibrated glass electrode at 25°C in water.

Suitable compound(s) according to group a) are sodium bisulfate, potassium bisulfate monosodium phosphate, and/or mixtures thereof. The preferred compound according to group a) is potassium bisulfate.

It is further preferred from the viewpoint of stabilization that the total concentration of the one or more compound(s) according to group a) in composition A is 0.1% by weight or more, preferably 0.25% by weight or more, still more preferably 0.5% by weight or more, calculated to the total weight of composition A.

It is further preferred from the viewpoint of dyeing intensity that the total concentration of the one or more compound(s) according to group a) in composition A is 15% by weight or less, preferably10% by weight or less, still more preferably 7.5% by weight or less, calculated to the total weight of composition A.

For attaining the above-mentioned effect, it is preferred that the total concentration of the one or more compound(s) according to group a) in composition A is in the range of 0.1% to 15% by weight, preferably in the range of 0.25% to 10% by weight, more preferably in the range of 0.5% to 7.5% by weight, calculated to the total weight of composition A.

The composition of the present invention further comprises one or more oxidative hair dye(s) as compound(s) according to group b).

Suitably, these oxidative hair dyes are one or more o-phenylenediamines, m-phenylenediamines, p-phenylenediamines, p-toluene-diamine, resorcinols, m-aminophenols, pyridines, naphtols, and/or their salt(s), and/or their mixtures.

It is preferred from the viewpoint of cosmetic acceptance, stability, and dyeing intensity that the one or more compound(s) according to group b) is/are p-phenylenediamine, 2-methoxy-methyl-p-phenylenediamine, p-toluene-diamine, hydroxyethyl-p-phenylene-diamine, resorcinol, 2-methylresorcinol, 4-chlororesorcinol, 2-amino-3-hydroxypyridine, 5-amino-6-chloro-o-cresol, 2,6-dihydroxy-3,4-dimethylpyridine, 2,6-dimethoxy-3,5-pyridine-diamine HCl, 5-amino-2,6-dimethoxy-3-hydroxypyridine, 6-methoxy-2-methylamino-3-aminopyridine, 2,3-diamino-6-methoxy-pyridine, 2,6-diaminopyridine, 1,3-bis(2,4-diamino-phenoxy)propane, and/or their salt(s), and/or their mixture(s).

The most preferred compound(s) according to group b) is/are 2-methoxy-methyl-p-phenylenediamine, resorcinol, 2-methylresorcinol, 4-chlororesorcinol, 2-amino-3-hydroxypyridine, 5-amino-6-chloro-o-cresol, 2,6-dihydroxy-3,4-dimethyl-pyridine, 2,6-dimethoxy-3,5-pyridine-diamine HCl, 5-amino-2,6-dimethoxy-3-hydroxypyridine, 6-methoxy-2-methylamino-3-aminopyridine, 2,3-diamino-6-methoxy-pyridine, 2,6-diaminopyridine, 1,3-bis(2,4-diamino-phenoxy)propane, and/or their salt(s), and/or their mixture(s).

It is preferred from the viewpoint of dyeing intensity that the total concentration of one or more compound(s) according to group b) is 0.1% by weight or more, preferably 0.25% by weight or more, still more preferably 0.5% by weight or more, furthermore preferably 1% by weight or more, calculated to the total weight of composition A.

It is preferred from the viewpoint of dyeing intensity that the total concentration of one or more compound(s) according to group b) is 50% by weight or less, preferably 30% by weight or less, still more preferably 20% by weight or less, furthermore preferably 10% by weight or less, calculated to the total weight of composition A.

For attaining the above-mentioned effects, it is preferred that the total concentration of one or more compound(s) according to group b) is in the range of 0.1% to 50% by weight, preferably in the range of 0.25% to 30% by weight, furthermore preferably in the range of 0.5% to 20% by weight, still more preferably in the range of 1% to 10% by weight, calculated to the total weight of composition A.

It is further preferred from the viewpoint of cosmetic convenience that the composition A further comprises one or more alkalizing agent(s) being solid at 25°C and atmospheric pressure.

Suitably, the composition A comprises at least one alkalizing agent being solid at 25°C and atmospheric pressure selected from one or more phosphate(s), one or more disilicate(s), one or more metasilicate(s), one or more carbonate(s), one or more bicarbonate(s), one or more Tris(hydroxymethyl)aminomethane, one or more silicate(s), and/or its/their salts, and/or their mixtures, as alkalizing agents. The preferred said alkalizing agents are one or more phosphate(s) or one or more metasilicate(s), and/or its/their salts, and/or their mixtures, still more preferably said alkalizing agents are sodium or potassium triphosphate and sodium metasilicate, and/or their mixtures.

It is preferred from the viewpoint of dyeing intensity that the composition A comprises said alkalizing agents at a total concentration of 1% by weight or more, more preferably at 5% by weight or more, still more preferably at 10% by weight or more, still more preferably at 15% by weight or more, calculated to the total weight of composition A.

It is preferred from the viewpoint of dyeing intensity that the composition A comprises said alkalizing agents at a total concentration of 75% by weight or less, more preferably at 70% by weight or less, calculated to the total weight of composition A.

For attaining the above-mentioned effects, it is preferred that the total concentration of alkalizing agents being solid at 25°C and atmospheric pressure in composition A is in the range of 1% to 75% by weight, more preferably 5% to 70% by weight, still more preferably in the range of 10% to 70% by weight, still more preferably in the range of 15% to 70% by weight, calculated to the total weight of the composition A.

It is further preferred from the viewpoint of cosmetic usability that the composition A further comprises one or more pulverulent excipients, preferably selected from organic/inorganic materials such as diatomaceous earth, cellulose, starch especially corn, tapioca, rice, wheat and potato, nylon powder, gypsum, sawdust and perlite, preferably said pulverulent excipient is cornstarch.

It is preferred that the total concentration of one or more pulverulent excipients in composition A is in the range of 20% to 70% by weight, preferably in the range of 25% to 50% by weight, more preferably in the range of 25% to 30% by weight, calculated to the total weight of the composition A.

For this aspect, it is preferred that composition A is a powder composition, a pellet or tablet composition.

It is further preferred that the total concentration of water in the composition A is 5% by weight or less, more preferably 1% by weight or less, still more preferably 0.5% by weight or less, calculated to the total weight of composition A, more preferably the composition A is anhydrous.

It is further preferred that the composition A further comprises one or more lipophilic compound(s) being liquid at 25°C and atmospheric pressure.

Suitable non-limiting examples of the one or more lipophilic compound(s) is/are isopropyl myristate (IPM), paraffin, squalane, phenyl trimethicone, and silicone, and/or their mixtures.

It is preferred that the total concentration of the lipophilic compound(s) as defined above is in the range of 0.1% to 20% by weight, more preferably in the range of 1% to 10% by weight, most preferably in the range of 2% to 6% by weight, calculated to the total weight of composition.

In another aspect of the present invention, preferably the composition A is a hair dyeing oil and the total concentration of lipophilic compounds as defined above is in the range of 20% to 50% by weight, more preferably in the range of 25% to 40% by weight, most preferably in the range of 25% to 30% by weight, calculated to the total weight of the composition A.

### Surfactants

Optionally, the oxidative dyeing composition according to the present invention may further comprise one or more surfactant(s), preferably selected from non-ionic surfactants, anionic surfactants, cationic surfactants, and/or amphoteric/zwitterionic surfactants, and/or their salt(s), and/or their mixtures, more preferably selected from anionic surfactants and/or their salt(s), from the viewpoint of improving wettability and mixability.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts.

Suitable examples are alkyl sulfate or preferably ethoxylated alkyl ether sulfate surfactants or mixtures thereof, and/or salts thereof, having an alkyl chain length of C₁₀ to C₂₂ and an ethoxylation degree from 1 to 50.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants, and/or their salts. Suitable examples are cetrimonium chloride and behentrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of the oxidative dyeing compositions, from the viewpoint of enhancing wettability of keratin fibers, and mixability with other compositions.

### Thickening polymers

From the viewpoint of cosmetic safety, it is further preferred that the oxidative hair dyeing composition comprises one or more thickening polymer(s).

The oxidative hair dyeing composition may comprise one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH ranges between 7 and 12 having a viscosity of at least 1,000 mPa•s measured at a polymer concentration of 1% by weight in water at 25°C determined by cone plate viscometry at 25°C under atmospheric conditions, calculated to the total weight of the composition, determined by cone-plate viscometry, suitably with a Brookfield viscometer with spindle #4 measured at 10 rpm for 1 min at 25°C and atmospheric conditions.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch-based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

Suitable synthetic anionic polymers are associative thickening polymers, such as acrylates/steareth-30 methacrylate copolymer.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers of the present invention are 0.1% by weight or more, more preferably 0.25% by weight or more, more preferably 0.5% by weight or more, calculated to the total weight of the oxidative hair dyeing composition, from the viewpoint of providing sufficient viscosity to the composition.

Preferably, the total concentration of thickening polymers of the present invention are 15% by weight or less, more preferably 12% by weight or more, furthermore preferably 10% by weight or less, calculated to the total weight of the oxidative hair dyeing composition, from the viewpoint of providing sufficient viscosity to the composition and cost of goods.

For attaining the above-mentioned effects, it is preferred that the total concentration of thickening polymers in the composition of the present invention is in the range of 0.1% to 15% by weight, preferably in the range of 0.25% to 12% by weight, more preferably in the range of 0.5% to 10% by weight, calculated to the total weight of the oxidative hair dyeing composition.

It is preferred from the viewpoint of cosmetic safety that the composition of the present invention, in case that it is aqueous, that it has a viscosity in the range of 1,000 mPas to 25,000 mPas, preferably 2,000 mPas to 20,000 mPas, more preferably in the range of 2,500 mPas to 17,500 mPas, determined by cone plate viscometry at 25°C under atmospheric conditions. A method for determining the viscometry is cone-plate viscometry, suitably with a Brookfield viscometer and spindle #4, measured at 10 rpm for 1 min at 25°C.

### Kit-of-parts

The present invention is also directed to a kit-of-parts comprising:
- the composition as defined above, and
- an aqueous composition B having a pH in the range of 1 to 6 and comprising one or more oxidizing agent(s), preferably hydrogen peroxide.

Preferably the total concentration of the hydrogen peroxide in composition B is in the range of 1% to 20% by weight, more preferably in the range of 2% to 15% by weight, most preferably in the range of 3% to 12% by weight, calculated to the total weight of the composition B.

It is preferred that the pH of composition B is in the range of 2 to 5, more preferably the pH is in the range of 2.5 to 4.5.

It is further preferred that the composition A as defined above and the composition B are kept separate until directly prior to the application onto keratin fibers.

### Method for dyeing keratin fibers

The present invention is also directed to a method for dyeing keratin fibers, preferably human keratin fibers, more preferably human hair, comprising the steps of:
i) mixing the composition A as defined above with a composition B as defined above to yield a ready-to-use composition having a pH in the range of 6 to 12,
ii) applying the ready-to-use composition onto keratin fibers and leaving it for a time period in the range of 1 min to 60 min,
iii) rinsing-off the keratin fibers and optionally shampooing the keratin fibers.

Preferably the pH of the ready-to-use composition in step (i) is in the range of 8 to 11, more preferably 8.5 to 10.5.

Further, it is preferred that the ready-to-use composition is left in step (ii) onto keratin fibers for a time period in the range of 5 to 45 min, more preferably 10 to 40 min.

### Use of compounds according to group a)

The present invention is also directed to the use of organic or inorganic salt(s) yielding an acidic solution at a concentration of 1% by weight in water at 25°C under atmospheric pressure as defined above for improving the storage stability of oxidative hair dyeing compositions comprising water at 10% by weight or less, calculated to the total weight of the oxidative hair dyeing composition.

The preferred inorganic salts are the ones as listed above for the composition A.

The following examples are to illustrate the invention, but not to limit it.

### Examples

**Table 1: The following examples were prepared by conventional mixing techniques.**

| **Ingredients** | | **Compositions** | | | | | | | | | | |
|---|---|---|---|---|---|---|---|---|---|---|---|---|
| | | **Comp. Ex. 1** | **Comp. Ex. 2** | **Comp. Ex. 3** | **Comp. Ex. 4** | **Comp. Ex. 5** | **Inv. Ex. 1** | **Inv. Ex. 2** | **Inv. Ex. 3** | **Inv. Ex. 4** | **Inv. Ex. 5** | **Inv. Ex. 6** |
| | | [% by weight] | | | | | | | | | | |
| - | Sodium triphosphate | 73.0 | 73.0 | 73.0 | 73.0 | 73.0 | 73.0 | 73.0 | 73.0 | 73.0 | 73.0 | 73.0 |
| b) | 2-methexy-methyl-p-phenylene-diamine sulfate | 2.28 | 2.28 | 0.25 | 0.25 | 1.14 | 2.28 | 2.28 | 0.25 | 0.25 | 0.25 | 1.14 |
| a) | KHSO₄ | - | - | - | - | - | 10.0 | 10.0 | 10.0 | 2.5 | 10.0 | 10.0 |
| b) | 4-chlororesorcinol | 1.32 | - | - | - | - | 1.32 | - | - | - | - | - |
| b) | 2-methylresorcinol | - | 1.13 | - | - | - | - | 1.13 | - | - | - | - |
| b) | 1,3-bis(2,4-diamino-phenoxy)-propane 4HCl | - | - | 0.44 | - | - | - | - | 0.44 | 0.44 | - | - |
| b) | 6-amino-o-cresol | - | - | - | 0.16 | - | - | - | - | - | 0.16 | - |
| b) | 2-amino-3-hydroxypyridine | - | - | - | - | 0.50 | - | - | - | - | - | 0.50 |
| - | Isopropyl myristate | 2.5 | 2.5 | 2.5 | 2.5 | 2.5 | 2.5 | 2.5 | 2.5 | 2.5 | 2.5 | 2.5 |
| - | Cornstarch | **Add to 100.00** | | | | | | | | | | |

| **Evaluation - Storage Stability** | | | | | | | | | | | | |
|---|---|---|---|---|---|---|---|---|---|---|---|---|
| Color Loss [ΔΔE] | | -5 | -11 | -7 | -18 | -11 | -4 | -3 | -3 | -1 | 0 | -1 |

### Discussion of Results

The inventive compositions (s. Table 1, Inv. Ex. 1-6) comprising potassium bisulfate, a representative of the salts yielding an acidic pH at 1% by weight in water, were stored for 1 month at the temperature of 50°C and showed the highest stability juxtaposed to the comparative compositions (s. Table 1, Comp. Ex. 1-5) stored for 1 month at the temperature of 50°C. To evaluate the storage effect with and without the salt, the color loss was measured. The inventive compositions (Inv. Ex. 1-6) showed little or almost none change in the ΔE values (s. Table 1) for different couplers tested, whereas the comparative compositions showed a strong decrease in color intensity.

### Methods

### Storage stability tests

To test the stability, the compositions (s. Table 1) were stored at 50°C for one month.

Goat Swatch hairstreaks (11 cm, 2 g per bundle) were applied the dyeing compositions of table 1 having been mixed prior to storage and after storage for 1 month with an oxidative composition comprising 6% by weight of hydrogen peroxide in a weight ratio of 1:10 to prepare ready-to-use compositions having a pH in the range of 9.5±0.5.

2 g of the ready-to-use compositions were applied onto the goat hairstreaks for 20 min at 40°C. The streaks were then rinsed off and dried. The colorimetric data were obtained with a color-difference meter (Datacolor Check II Plus) in the CIE colorimetric system (L*, a*, b*). Color differences (ΔE) were calculated by the following formula. $Δ E = \sqrt{{\left({L}_{1}^{∗}-{L}_{0}^{∗}\right)}^{2} + {\left({a}_{1}^{∗}-{a}_{0}^{∗}\right)}^{2} + {\left({b}_{1}^{∗}-{b}_{0}^{∗}\right)}^{2}}$ and $ΔΔE = {ΔE}_{after storage} - {ΔE}_{befor storage} .$

## Claims

1. An oxidative hair dyeing composition A comprising:
a) One or more organic or inorganic salt(s) yielding an acidic solution at a concentration of 1% by weight in water at 25°C under atmospheric pressure,
b) One or more oxidative hair dye(s),
Wherein the composition A comprises water at 10% by weight or less, calculated to the total weight of composition A.

2. The composition according to claim 1 **characterized in that** the one or more compound(s) according to group a) are selected from sodium bisulfate, potassium bisulfate, monosodium phosphate, and/or mixtures thereof.

3. The composition according to any of the claims 1 and/or 2 **characterized in that** the total concentration of the one or more compound(s) according to group a) is in the range of 0.1% to 15% by weight, preferably in the range of 0.25% to 10% by weight, more preferably in the range of 0.5% to 7.5% by weight, calculated to the total weight of composition A.

4. The composition according to any of the preceding claims **characterized in that** the one or more compound(s) according to group b) is/are one or more o-phenylenediamines, m-phenylenediamines, p-phenylenediamines, p-toluene-diamine, resorcinols, m-aminophenols, pyridines, naphtols, and/or their salt(s), and/or their mixtures, preferably the one or more compound(s) according to group b) is/are p-phenylenediamine, 2-methoxy-methyl-p-phenylenediamine, p-toluene-diamine, hydroxyethyl-p-phenylene-diamine, resorcinol, 2-methylresorcinol, 4-chlororesorcinol, 2-amino-3-hydroxypyridine, 5-amino-6-chloro-o-cresol, 2,6-dihydroxy-3,4-dimethyl-pyridine, 2,6-dimethoxy-3,5-pyridine-diamine HCl, 5-amino-2,6-dimethoxy-3-hydroxypyridine, 6-methoxy-2-methylamino-3-aminopyridine, 2,3-diamino-6-methoxy-pyridine, 2,6-diaminopyridine, 1,3-bis(2,4-diamino-phenoxy)propane, and/or their salt(s), and/or their mixture(s), more preferably the one or more compound(s) according to group b) is/are 2-methoxy-methyl-p-phenylenediamine, resorcinol, 2-methylresorcinol, 4-chlororesorcinol, 2-amino-3-hydroxypyridine, 5-amino-6-chloro-o-cresol, 2,6-dihydroxy-3,4-dimethylpyridine, 2,6-dimethoxy-3,5-pyridine-diamine HCl, 5-amino-2,6-dimethoxy-3-hydroxypyridine, 6-methoxy-2-methylamino-3-aminopyridine, 2,3-diamino-6-methoxy-pyridine, 2,6-diaminopyridine, 1,3-bis(2,4-diamino-phenoxy)propane, and/or their salt(s), and/or their mixture(s).

5. The composition according to any of the preceding claims **characterized in that** the total concentration of one or more compound(s) according to group b) is in the range of 0.1% to 50% by weight, preferably in the range of 0.25% to 30% by weight, furthermore preferably in the range of 0.5% to 20% by weight, still more preferably in the range of 1% to 10% by weight, calculated to the total weight of composition A.

6. The composition according to any of the preceding claims **characterized in that** the composition A further comprises one or more alkalizing agent(s) being solid at 25°C and atmospheric pressure, preferably the composition A comprises at least one alkalizing agent(s) being solid at 25°C and atmospheric pressure selected from one or more phosphate(s), one or more disilicate(s), one or more metasilicate(s), one or more carbonate(s), one or more bicarbonate(s), one or more Tris(hydroxymethyl)aminomethane, one or more silicate(s), and/or its/their salts, and/or their mixtures, preferably said one or more alkalizing agent(s) is/are one or more phosphate(s) or one or more metasilicate(s), and/or its/their salts, and/or their mixtures, still more preferably said alkalizing agents are sodium or potassium triphosphate and sodium metasilicate, and/or their mixtures.

7. The composition according to claim 6 **characterized in that** the total concentration of alkalizing agents being solid at 25°C and atmospheric pressure in composition A is in the range of 1% to 75% by weight, more preferably 5% to 70% by weight, still more preferably in the range of 10% to 70% by weight, still more preferably in the range of 15% to 70% by weight, calculated to the total weight of the composition A.

8. The composition according to any of the preceding claims **characterized in that** the composition A further comprises one or more pulverulent excipients, preferably selected from organic/inorganic materials such as diatomaceous earth, cellulose, starch especially corn, tapioca, rice, wheat and potato, nylon powder, gypsum, sawdust and perlite, preferably said pulverulent excipient is cornstarch.

9. The composition according to claim 8 **characterized in that** the total concentration of one or more pulverulent excipients is in the range of 20% to 70% by weight, preferably in the range of 25% to 50% by weight, more preferably in the range of 25% to 30% by weight, calculated to the total weight of the composition.

10. The composition according to any of the preceding claims **characterized in that** the total concentration of water in the composition A is 5% by weight or less, more preferably 1% by weight or less, still more preferably 0.5% by weight or less, calculated to the total weight of composition A, more preferably the composition A is anhydrous.

11. The composition according to any of the preceding claims **characterized in that** the composition A is a powder composition, or a pellet or tablet composition.

12. The composition according to any of the claims 1 to 10 **characterized in that** the composition A further comprises one or more lipophilic compound(s) being liquid at 25°C and atmospheric pressure, and preferably the composition A is an oxidative hair dyeing oil composition.

13. A kit-of-parts comprising:
- the composition as defined in any of the claims 1 to 12, and
- an aqueous composition B having a pH in the range of 1 to 6 and comprising one or more oxidizing agent(s), preferably hydrogen peroxide.

14. A method for dyeing keratin fibers, preferably human keratin fibers, more preferably human hair, comprising the steps of:
i) mixing the composition A as defined in any of the claims 1 to 12 with a composition B as defined in claim 13 to yield a ready-to-use composition having a pH in the range of 6 to 12,
ii) applying the ready-to-use composition onto keratin fibers and leaving it for a time period in the range of 1 min to 60 min,
iii) rinsing-off the keratin fibers and optionally shampooing the keratin fibers.

15. Use of organic or inorganic salt(s) yielding an acidic solution at a concentration of 1% by weight in water at 25°C under atmospheric pressure as defined in any of the claims 1 to 12 for improving the storage stability of oxidative hair dyeing compositions comprising water at 10% by weight or less, calculated to the total weight of the oxidative hair dyeing composition.
